# EUROPÄISCHE PATENTSCHRIFT

(11) **EP 0 662 816 B1**
(45) Veröffentlichungstag und Bekanntmachung des Hinweises auf die Patenterteilung: **08.01.1997**
(21) Anmeldenummer: 93920768.4
(22) Anmeldetag: 20.09.1993
(51) Int. Cl.: A61K 7/06, A61K 7/50

(54) **HAARNACHBEHANDLUNGSMITTEL**
HAIR AFTER-TREATING AGENTS
COMBINAISON D'UN SHAMPOING/APRES-SHAMPOING

(30) Priorität: 29.09.1992 DE 4232512; 29.09.1992 DE 4232506; 13.10.1992 DE 4234405; 13.10.1992 DE 4234413; 27.05.1993 DE 4317576
(43) Veröffentlichungstag der Anmeldung: 19.07.1995
(73) Patentinhaber: Henkel Kommanditgesellschaft auf Aktien, 40191 Düsseldorf (DE)
(72) Erfinder: KAHRE, Jörg, D-40789 Monheim (DE); MÜLLER-KIRSCHBAUM, Thomas, D-42699 Solingen (DE); HENSEN, Hermann, D-42781 Haan (DE); TESMANN, Holger, D-41363 Jüchen (DE); GOEBELS, Dagmar, D-40591 Düsseldorf (DE)
(86) Internationale Anmeldenummer: EP9302535
(87) Internationale Veröffentlichungsnummer: WO9407458

(56) Entgegenhaltungen:
- EP-A- 0 217 274
- EP-A- 0 283 817
- EP-A- 0 473 349
- EP-A- 0 502 616
- GB-A- 2 155 472

## Beschreibung

Die Erfindung betrifft die Verwendung von Mitteln mit speziellen Wirkstoffkombinationen zum Reinigen und Pflegen von Keratinfasern, insbesondere von Haaren.

Die Reinigung und Pflege der Haare ist ein wichtiger Bestandteil der menschlichen Körperpflege. Sowohl die Reinigung und Pflege der Haare beispielsweise mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein. Weiterhin können die Haare einen erhöhten Spliß aufweisen oder aufgrund elektrostatischer Aufladung "fliegen".

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert. Weiterhin wurden in jüngster Zeit verstärkte Anstrengungen zur Suche nach Wirkstoffen oder Wirkstoffkombinationen unternommen, die sich direkt in die verschiedenen Haarbehandlungsmittel einarbeiten lassen und somit den zusätzlichen Nachbehandlungsschritt überflüssig machen.

Zusätze von kationischen Polymeren zu Haarbehandlungsmitteln führen in der Regel zu einer verbesserten Naß- und Trockenkämmbarkeit; Zusätze von amphoteren Polymeren zu einer stark verbesserten Naßkämmbarkeit, während die Trockenkämmbarkeit meist nur wenig beeinflußt wird.

Während die Verbesserung der Naßkämmbarkeit, d.h. eine Erniedrigung der Naßkämmarbeit, in allen Fällen gewünscht wird, sind die Verhältnisse bei der Trockenkämmbarkeit komplizierter. Niedrige Kämmarbeits-Werte charakterisieren zwar eine Verbesserung der Kämmbarkeit; wird die Kämmarbeit aber zu sehr erniedrigt, so verliert das Haar Fülle und Halt, so daß sich im Extremfall bestimmte Frisuren nicht mehr aufbauen lassen. Daher kann, vor allem bei komplexeren Frisuren, eine in bestimmten Grenzen erhöhte Trockenkämmarbeit durchaus erwünscht sein, um den Halt der Frisur zu verbessern. Diese ist in vielen Fällen aber mit einer erhöhten elektrostatischen Aufladung der Haare verbunden, was zum unerwünschten Phänomen des "Fliegens" der Haare führt.

Es besteht daher nach wie vor ein Bedarf an verbesserten Wirkstoffen und Wirkstoffkombinationen für die Nachbehandlung von Haaren bzw. zum Einbau in bekannte Haarreinigungs- und Pflegemittel.

Es wurde nun überraschenderweise gefunden, daß Wirkstoffkombinationen aus bestimmten Polymeren (A), speziellen Alkylpolyglykosiden (B) und Fett- oder Wachsstoffen (C) einen erheblichen Beitrag zur Lösung dieser Problematik leisten können. Die damit behandelten Haare weisen eine sehr gute Naßkämmbarkeit auf; die Trockenkämmbarkeit liegt in einem für den Halt der Frisur sehr günstigen Bereich, ohne daß in nennenswertem Umfang elektrostatische Aufladung, und damit ein "Fliegen" der Haare beobachtet wird.

Gegenstand der Erfindung ist daher die Verwendung eines wäßrigen Mittels, gekennzeichnet durch einen Gehalt an
a) Polymeren (A) , ausgewählt aus der Gruppe der kationischen, amphoteren, zwitterionischen und nichtionischen Polymeren,
b) Alkylpolyglykosiden (B) der allgemeinen Formel (I)

   RO-(Z)ₓ (I)

   in denen
   - R: steht für einen Alkylrest mit 6 bis 22 Kohlenstoffatomen,
   - Z: für einen Mono- oder Oligosaccharid,
   - x: für eine Zahl von 1,1 bis 5,
   oder deren Anlagerungsprodukten mit 1 bis 10 Molekülen Ethylenoxid
   und/oder Propylenoxid sowie
c) Fettalkoholen (C)
zum Reinigen oder Pflegen von Keratinfasern, insbesondere menschlichen Haaren.

Alle drei Wirkstoffklassen sind dem Fachmann geläufige Bestandteile von Haarbehandlungsmitteln.

Ebenfalls bekannt sind Kombinationen von jeweils zwei dieser drei Wirkstoffklassen. So ist aus einem Beispiel der DE-OS 32 16 687 ein Shampoonierungsmittel bekannt, das neben einem kationischen Polymeren (Gafquat 755) einen Glucosid-alkylether (Triton CG 110) sowie polyethoxylierten Laurylalkohol enthält. Gleichfalls Kombinationen aus kationischen Polymeren und Alkylsacchariden sind aus der EP-A1-337 354 bekannt.

Schließlich sind aus Beispielen der nicht vorveröffentlichten deutschen Patentanmeldungen P 42 32 512.9, P 42 32 506.4, P 42 34 413.1 und P 42 34 405.0 Kombinationen von Alkylglykosiden, kationischen bzw. zwitterionischen Polymeren und Fettalkoholen bzw. Mono- und Triglyceriden in Dauerwellmitteln bekannt. Aus Beispielen der P 42 32 506.4 sind solche Kombinationen ebenfalls für Shampoos und Tönungsshampoos bekannt.

Diesem Stand der Technik sind aber keine Hinweise auf die vorteilhaften Effekte zu entnehmen, die durch die Verwendung der erfindungsgemäßen Wirkstoff-Dreierkombination in Mitteln zur Reinigung und Pflege von Keratinfasern, insbesonderen Haaren, erzielt werden. Unter "Mitteln zur Pflege" im Sinne dieser Anmeldung sind nur solche Mittel zu verstehen, die der (Wieder-)herstellung von natürlichen Eigenschaften der Haare, wie gute Kämmbarkeit, Halt der Frisur, Fülle des Haares usw. dienen. Ausdrücklich nicht unter diesen Begriff fallen Dauerwellmittel, Haarfärbemittel, Tönungsshampoos und Haarfestiger, die Veränderung im Aussehen des Haares zu dekorativen Zwecken bewirken.

Die erfindungsgemäß verwendeten Mittel enthalten bevorzugt 0,1 bis 3 Gew.-% an Polymeren (A), 0,01 bis 10 Gew.-% an Alkylpolyglykosiden (B) und 0,5 bis 20 Gew.-% an Fettalkoholen (C), jeweils bezogen auf das gesamte Mittel.

Die erste Komponente der erfindungesgemäßen Wirkstoffkombination ist ausgewählt aus der Gruppe der kationischen, amphoteren, zwitterionischen und nichtionischen Polymeren.

Die erfindungsgemäß verwendbaren kationischen Polymeren enthalten innerhalb des Polymergerüstes kationische Gruppen. Diese Gruppen können Teil der Polymerkette sein; sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliniumgruppen.

Befinden sich die ionischen Gruppen in den Seitenketten, so sind die Polymeren aus Verbindungen aufgebaut, die neben mindestens einer kationischen Gruppe mindestens eine polymerisierbare Gruppe enthalten und frei sind von anionischen Gruppen.

Die polymerisierbare Gruppe ist bevorzugt eine Vinylgruppe. Es sind jedoch auch kationische Polymerisate verwendbar, bei denen die Polymerhauptkette beispielsweise aus Glykosiden aufgebaut ist oder Proteincharakter hat.

Erfindungsgemäß ebenfalls bevorzugt sind kationische Copolymere, die neben den kationischen Monomeren noch mindestens ein nichtionisches Monomer enthalten. Geeignete nichtionische Monomere sind beispielsweise Vinylpyrrolidon, Vinylacetat, Acrylamid, Methacrylamid, Methylacrylat, Ethylacrylat, Methylmethacrylat und Ethylmethacrylat. Vinylpyrrolidon ist ein besonders bevorzugtes nichtionisches Monomer.

Eine Reihe von für die Haarpflege geeigneten kationischen Polymeren sind dem Fachmann bekannt und als Handelsprodukte erhältlich.

Beispiele für solche Polymeren sind:
- quaternierte Cellulose-Derivate, wie sie unter den Bezeichnungen Celquat^{R} und Polymer JR^{R} im Handel erhältlich sind. Die Verbindungen Celquat H 100, Celquat L 200 und Polymer JR^{R}400 sind bevorzugte quaternierte Cellulose-Derivate.
- quaternierte Guar-Derivate, wie sie unter den Bezeichnungen Cosmedia Guar^{R} und Jaguar^{R} im Handel erhältlich sind. Bevorzugte Guar-Derivate sind beispielsweise Cosmedia Guar^{R} C-261 und Jaguar^{R} C 13-S.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{R}734 und Gafquat^{R}755 im Handel erhältlich.
- Copolymerisate des Vinylpyrrolidons mit Vinylimidazoliummethochlorid, wie sie unter der Bezeichnung Luviquat^{R} angeboten werden.
- Polymere Dimethyldiallylammoniumsalze und deren Copolymere mit Estern und Amiden von Acrylsäure und Methacrylsäure. Die unter den Bezeichnungen Merquat^{R}100 (Poly(dimethyldiallylammoniumchlorid)) und Merquat^{R}550 (Dimethyldiallylammoniumchlorid-Acrylamid-Copolymer) im Handel erhältlichen Produkte sind Beispiele für solche kationischen Polymere.
- Kationisch derivatisierte Proteinhydrolysate, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Die Proteine, die als Ausgangstoffe für die Proteinhydrolysate dienen, können sowohl tierischer als auch pflanzlicher Herkunft sein. Übliche Ausgangsstoffe sind beispielsweise Keratin, Kollagen, Elastin, Sojaprotein, Milchprotein, Weizenprotein, Seidenprotein und Mandelprotein. Durch die Hydrolyse entstehen Stoffmischungen mit Molmassen im Bereich von ca. 100 bis ca. 50 000 Dalton. Übliche mittlere Molmassen liegen in einem Bereich von etwa 500 bis etwa 5000 Dalton. Nähere Einzelheiten über kationische Derivatisierung können u.a. der japanischen Patentanmeldung 77/73485 (Chemical Abstracts Referat 90:174508v) entnommen werden.
   Vorteilhafterweise enthalten die kationisch derivatisierten Proteinhydrolysate eine oder zwei lange Alkylketten mit 8 bis 22 C-Atomen und entsprechend zwei oder eine kurze Alkylkette mit 1 bis 4 C-Atomen. Verbindungen, die eine lange Alkylkette enthalten, sind bevorzugt.
   Bevorzugte Verbindungen (A) sind Substanzen der Formel (I), 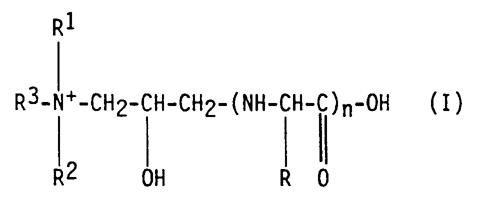 in der R für die Seitenketten der Aminosäuren des Proteins, R¹ und R² unabhängig voneinander für Alkylketten mit 1 bis 4 C-Atomen und R³ für eine Alkylkette mit 8 bis 22 C-Atomen steht.
   Ein auf dem Markt erhältliches Produkt ist Lamequat^{R}L (Chemische Fabrik Grünau). Es hat die Struktur 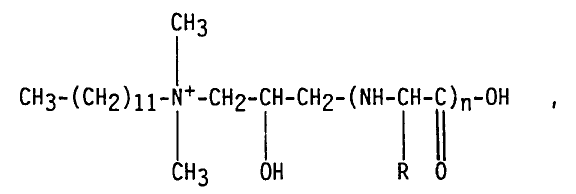 in der R für die Seitenketten der Aminosäuren des Kollagens steht. Eine Bezeichnung analog CTFA ist Lauryldimonium Hydroxypropylamino Hydrolyzed Collagene.
- Polymere Kondensationsharze von Polyolen und Polyaminen, wie beispielsweise Polyglykol-Polyamin-Kondensationsharze, die unter der CTFA-Bezeichnung PEG-15 Cocopolyamine bekannt sind. Im Handel ist beispielsweise das Produkt Polyquart^{R}H 81 (Henkel) erhältlich.

Unter "amphoteren Polymeren" sollen im Sinne der Erfindung Polymere verstanden werden, die im Molekül sowohl freie Aminogruppen als auch freie -COOH- oder -SO₃H-Gruppen enthalten und zur Ausbildung innerer Salze befähigt sind. Unter "zwitterionischen Polymeren" werden solche Polymeren verstanden, die im Molekül quartäre Ammoniumgruppen und -COO⁻- oder -SO₃⁻-Gruppen enthalten.

Beispiele für erfindungsgemäß einsetzbare amphotere Polymere sind die unter den Bezeichnung Amphomer^{R} und Amphomer^{R} LV-71 erhältlichen Acrylharze, die Copolymere aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellen.

Weitere erfindungsgemäß einsetzbare amphotere oder zwitterionische Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Besonders bevorzugt werden zwitterionische Polymere, die sich im wesentlichen zusammensetzen aus
(α) Monomeren mit quartären Ammoniumgruppen der allgemeinen Formel (I),

   R¹-CH=CR²-CO-X-(CₙH₂ₙ)-N⁽⁺⁾R³R⁴R⁵ A⁽⁻⁾ (I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, X eine NH-Gruppe oder ein Sauerstoffatom, n eine ganze Zahl von 2 bis 5 und A⁽⁻⁾ das Anion einer organischen oder anorganischen Säure ist
   und
(β) monomeren Carbonsäuren der allgemeinen Formel (II),

   R⁶-CH=CR⁷-COOH (II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen.

Ganz besonders bevorzugt sind solche Polymeren auf Basis von Monomeren des Typs (α), bei denen R³, R⁴ und R⁵ Methylgruppen sind, X eine NH-Gruppe und A⁽⁻⁾ ein Halogenid-, Methoxysulfat- oder Ethoxysulfat-Ion ist; Acrylamidopropyltrimethylammoniumchlorid und Metacrylamidopropyltrimethylammoniumchlorid sind besonders bevorzugte Monomere (α). Als Monomeres (β) für die genannten Polymeren wird bevorzugt Acrylsäure oder ein Alkalisalz der Acrylsäure, insbesondere das Natriumsalz, verwendet.

Weiterhin sind solche zwitterionischen Polymere bevorzugt, bei denen die Zahl der Monomeren vom Typ (α) größer als die Zahl der Monomeren vom Typ (β) ist. Monomerenverhältnisse (α):(β) größer als 1,5 sind besonders bevorzugt.

Ebenfalls bevorzugte zwitterionische Polymerisate sind Polysiloxan-Polyorganobetain-Copolymere.

Geeignete nichtionogene Polymere sind beispielsweise:
- Polyvinylpyrrolidone, beispielsweise die unter den Bezeichnungen Luviskol^{R} K 30 und Luviskol^{R} K 90 (BASF) erhältlichen Produkte.
- Vinylpyrrolidon/Vinylacetat-Copolymere, wie sie beispielsweise unter dem Warenzeichen Luviskol^{R} (BASF) vertrieben werden. Luviskol^{R} VA 64, Luviskol^{R} VA 73 und Luviskol^{R} VA 37 sind bevorzugte nichtionogene Polymere; Luviskol^{R} VA 37 ist besonders bevorzugt.
- Vinylpyrrolidon/Dimethylaminoethylmethacrylat/Vinylcaprolactam-Terpolymere, wie sie beispielsweise unter der Bezeichnung Copolymer VC-713 (GAF) erhältlich sind.

Unter den Polymeren (A) sind amphotere und zwitterionische Polymere bevorzugt. Als erfindungsgemäß ganz besonders gut verwendbar haben sich zwitterionische Polymere erwiesen, die aus mindestens einem Monomertyp mit kationischem Charakter und mindestens einem Monomertyp mit anionischem Charakter aufgebaut sind.

Die zweite Komponente der erfindungsgemäßen Wirkstoffkombination stellen Alkylpolyglykoside gemäß Formel (I) dar.

Diese Verbindungen gemäß Formel (I) sind durch folgende Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten. Auch diese Produkte stellen üblicherweise keine einheitlichen Verbindungen dar, sondern weisen in Abhängigkeit von dem gewählten Ethoxylierungsverfahren eine entsprechende Homologenverteilung auf. Solche alkoxylierten Verbindungen können beispielsweise dadurch erhalten werden, daß zur Synthese der Alkylpolyglykoside ethoxylierte Fettalkohole verwendet werden.

Überraschenderweise hat sich herausgestellt, daß bereits vergleichsweise geringe Mengen an Komponente (B) ausreichen, damit die erfindungsgemäßen Effekte auftreten. Es kann daher bevorzugt sein, Komponente (B) lediglich in Mengen von 0,1 bis 0,9 Gew.-%, bezogen auf das gesamte Mittel, zu verwenden.

Die dritte Komponente der erfindungsgemäßen Wirkstoffkombination sind die Fettalkohole. Besonders bevorzugt sind gesättigte und ungesättigte, lineare und verzweigte Fettalkohole mit 8 bis 24 Kohlenstoffatomen. Zu den bevorzugten Fettalkoholen gehören Decylalkohol, Laurylalkohol, Myristylalkohol, Cetylalkohol, Stearylalkohol, Oleylalkohol, Isostearylalkohol und Hydroxystearylalkohol.

Neben der erfindungsgemäßen Wirkstoffkombination können die erfindungsgemäß verwendbaren Mittel alle in solchen Mitteln üblichen Bestandteile enthalten. Handelt es sich bei den erfindungsgemäßen Mittel um Haarreinigungsmittel, so enthalten sie üblicherweise oberflächenaktive Verbindungen. Je nach Formulierung können die Mittel dann anionische, zwitterionische, ampholytische, kationische oder nichtionogene Tenside enthalten.

Als anionische Tenside eignen sich in erfindungsgemäßen Haarbehandlungsmitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 10 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Als kationische Tenside können in den erfindungsgemäßen Haarbehandlungsmitteln insbesondere quartäre Ammoniumverbindungen wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid enthalten sein. Weiterhin können als kationische Tenside sogenannte Esterquats (z. B. Stepantex^{R} VS 90, Dehyquart^{R} AU 36 und AU 56) sowie Amidoamine (z. B. Tegoamid^{R} S 18) eingesetzt werden.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- Glycerinmono- und -diester und Sorbitanmono- und -diester von gesättigten und ungesättigten C₈-C₂₂-Fettsäuren und deren Ethylenoxidanlagerungsprodukte und
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein, insbesondere, wenn es sich um ethoxylierte Fettalkohole handelt, die gleichzeitig als Verdickungsmittel dienen.

Vorzugsweise enthalten die erfindungsgemäßen Zubereitungen die oberflächenaktiven Verbindungen A in Mengen von 0,5 bis 20 Gew.-%, bezogen auf die jeweilige Zubereitung.

Besonders bevorzugt werden die erfindungsgemäßen Wirkstoffkombinationen in Haarnachbehandlungsmitteln verwendet, d.h. Mitteln, die nach einer Haarwäsche oder einer anderen Haarbehandlung, wie Dauerwellen oder Färbung, eingesetzt werden. Bei solchen Mitteln kann es bevorzugt sein, diese frei von ionischen, insbesondere von kationischen und anionischen, Tensiden zu formulieren.

Weitere übliche Bestandteile der erfindungsgemäß verwendeten Mittel können sein:
- Verdickungsmittel wie Agar-Agar, Guar-Gum, Alginate und Xanthan-Gum oder Ester aus ethoxylierten Polyolen und Fettsäuren wie beispielsweise Polyglyceryl(2)polyoxyethylen(4)stearat,
- Strukturanten wie Glucose und Maleinsäure,
- Proteinhydrolysate, insbesondere Elastin-, Kollagen-, Keratin-, Milcheiweiß-, Sojaprotein-, Mandel- und Weizenproteinhydrolysate sowie deren Kondensationsprodukte mit Fettsäuren
- Parfümöle, Dimethylisosorbid und Cyclodextrine,
- Lösungsvermittler, wie Ethanol, Isopropanol, Ethylenglykol, Propylenglykol, Glycerin und Diethylenglykol sowie ethoxylierte Fettalkohole,
- Farbstoffe,
- Antischuppenwirkstoffe wie Piroctone Olamine und Zink Omadine,
- Substanzen zur Einstellung des pH-Wertes wie Citronensäure/Natriumcitrat-Puffer,
- Wirkstoffe wie Panthenol, Allantoin, Pyrrolidoncarbonsäuren, Pflanzenextrakte und Vitamine,
- Lichtschutzmittel,
- Konsistenzgeber wie Zuckerester, Polyolester oder Polyolalkylether,
- Überfettungsmittel wie polyethoxylierte Lanolinderivate, Lecithinderivate und Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Metallseifen, wie beispielsweise Zink- oder Aluminiumstearat,
- Trübungsmittel wie Latex,
- Perlglanzmittel wie Ethylenglykolmono und -distearat,
- Treibmittel wie Propan-Butan-Gemische, N₂O, Dimethylether und Luft sowie
- Antioxidantien.

Die erfindungsgemäß verwendbaren Mittel können als Lotion, Emulsion, Mikroemulsion, Lösung, Creme oder Gel formuliert sein. Die Formulierung als Lotion, Emulsion oder Mikroemulsion mit einem Wassergehalt von 50 bis 90 Gew.-%, bezogen auf das gesamte Mittel, kann bevorzugt sein. Zur Einstellung der gewünschten Viskosität der Formulierung werden bevorzugt ethoxylierte Fettalkohole mit eingeengter Homologenverteilung und Ester aus ethoxylierten Polyolen und Fettsäuren, gewünschtenfalls auch Metallseifen, verwendet.

In einer weiteren bevorzugten Ausführungsform können die Mittel in Form von Schaumaerosolen konfektioniert werden, die mit einem verflüssigten Gas wie z. B. Propan-Butan-Gemischen, Stickstoff, CO₂, Luft, N₂O, Dimethylether, Fluorchlor- und Chlorkohlenwasserstofftreibmitteln oder Gemischen davon in Aerosolbehältern mit Schaumventil abgefüllt werden.

### Beispiele

### I. Bestimmung von Naß- und Trockenkammbarkeit sowie elektrostatischer Aufladung

### Untersuchungsmethoden

Den Untersuchungen zur Kämmbarkeit wurde die Methode gemäß J. Soc. Cosm. Chem. 1973 [24] 782 zugrundegelegt.

Es wurde jeweils die Kämmarbeit an braunem Haar (Alkinco #6634, Strähnenlänge 12 cm, Strähnenmasse 1 g) untersucht. Es handelte sich um leicht vorgeschädigte ("mediumblondierte") Haare, wie sie etwa beim durchschnittlichen Anwender zu erwarten sind. Dabei werden die Haare 30 Minuten lang mit einer 6%-igen H₂O₂-Lösung behandelt, die mit Ammoniak auf einen pH-Wert von 9,4 eingestellt wurde. Nach der Nullmessung wurden die Strähnen mit 1g zu prüfenden Zusammensetzung / g Haare getränkt. Nach 5 Minuten Einwirkzeit wurden die Strähnen 1 Minute unter fließendem Wasser (1 l/min, 38 °C) ausgespült. Zur Bestimmung der Naßkämmarbeit wurden die Strähnen dann erneut vermessen. Zur Bestimmung der Trockenkämmarbeit wurden die Strähnen zunächst jeweils 12 Stunden bei 30 °C und einer relativen Luftfeuchtigkeit von 20% getrocknet und dann vermessen.
Die Messung der Trockenkämmarbeit erfolgte dabei unter Zulassung der elektrostatischen Aufladung, die parallel zur Trockenkämmbarkeit bestimmt wurde. Die Messung der elektrostatischen Aufladung erfolgte über den Ladungsabgriff an einem doppelten Faraday-Käfig nach Ausführung von 10 Kämmungen.

### Ergebnisse

Die Zusammensetzung der untersuchten Mischungen sowie die Ergebnisse der Messungen sind in Tabelle 1 zusammengestellt. Die Werte stellen jeweils den Mittelwert von 20 Messungen mit 20 verschiedenen Strähnen dar und sind auf den Wert der Nullmessung bezogen; ihre statistische Sicherheit betrug 99,0 bzw. 99,99%.

**Tabelle 1**

| [Mengenangaben in Gewichtsteilen] | | | | |
|---|---|---|---|---|
| Komponente / Mischung | V1 | E1 | V2 | E2 |
| Cetyl/Stearylalkohol (1:1) | 3,2 | 3,2 | 3,2 | 3,2 |
| Plantaren^{R}1200¹ | - | 1,0 | - | 1,0 |
| Polyquart^{R}H 81² | 2,0 | 2,0 | - | - |
| Acrylamidopropyltrimethylammonium-chlorid/Acrylsäure (Massenverhältnis 70:30)-Copolymeres, mit Natronlauge neutralisiert | - | - | 1,0 | 1,0 |
| Parfumöl | 0,1 | 0,1 | 0,1 | 0,1 |
| Cetyl/Stearylalkohol + 20 EO | 0,8 | 0,8 | 0,8 | 0,8 |
| Wasser | <---------ad 100 ---------> | | | |
| Naßkämmarbeit [%] | 48 | 41 | 40 | 46 |
| Trockenkämmarbeit [%] | 152 | 218 | 307 | 302 |
| Elektrostatische Aufladung [%] | 120 | 162 | 205 | 156 |

| | | | | |
|---|---|---|---|---|
| ¹ C₁₂₋₁₆-Alkyl-polyglucosid (Polymerisationsgrad 1,4; ca. 50 % Aktivsubstanz; CTFA-Bezeichnung: Lauryl Polyglycose) (HENKEL CORP.) | | | | |
| ² Polyglykol-Polyamin-Kondensationsharz auf Basis PEG-Epichlorhydrin-Dipropylentriamin-Laurylamin (ca. 50 % Aktivsubstanz; CTFA-Bezeichnung: PEG-15-Coco Polyamine) (HENKEL) | | | | |

### II. Rezepturbeispiele

Alle Mengenangaben in den Rezepturbeispielen sind Gewichtsprozent.

### 1. Spülung

| | |
|---|---|
| Cetyl/Stearylalkohol | 3,2 |
| Plantaren^{R}2000³ | 1,0 |
| Acrylamidopropyltrimethylammoniumchlorid/Acrylsäure-Copolymeres, mit Natronlauge neutralisiert (P1 gemäß DE 39 29 973) | 1,0 |
| Parfümöl | 0,2 |
| Cetyl/Stearylalkohol + 20 EO | 1,2 |
| Sorbinsäure | 0,4 |
| Wasser | ad 100 |

| | |
|---|---|
| ³ C₈₋₁₆-Alkyl-polyglucosid (Polymerisationsgrad 1,4; ca. 50 % Aktivsubstanz; CTFA-Bezeichnung: Decyl Polyglycose) (HENKEL) | |

### 2. Spülung

| | |
|---|---|
| Lanette^{R}16⁴ | 3,2 |
| Plantaren^{R}2000 | 2,0 |
| Acrylamidopropyltrimethylammoniumchlorid/Acrylsäure-Copolymeres, mit Natronlauge neutralisiert (P1 gemäß DE 39 29 973) | 1,0 |
| Cutina^{R}GMS⁵ | 4,0 |
| Parfümöl | 0,1 |
| PHB-Ester | 0,3 |
| Wasser | ad 100 |

| | |
|---|---|
| ⁴ Cetylalkohol (CTFA-Bezeichnung: Cetyl Alcohol) (HENKEL) | |
| ⁵ Glycerinmonostearat (CTFA-Bezeichnung: Glyceryl Stearat) (HENKEL) | |

### 3. Spülung

| | |
|---|---|
| Lanette^{R}16 | 3,2 |
| Plantaren^{R}2000 | 2,0 |
| Cosmedia^{R} Guar C 261⁶ | 1,0 |
| Parfümöl | 0,2 |
| Eumulgin^{R}HRE 40⁷ | 0,3 |
| PHB-Ester | 0,3 |
| Wasser | ad 100 |

| | |
|---|---|
| ⁶ Guarhydroxypropyltrimethylammoniumchlorid (90 % Aktivsubstanz; CTFA-Bezeichnung: Guar Hydroxypropyl Trimonium Chloride) (HENKEL) | |
| 7 gehärtetes Rizinusöl mit 40 EO (CTFA-Bezeichnung: PEG-40 Hydrogenated Castor Oil) (HENKEL) | |

### 4. Shampoo

| | |
|---|---|
| Texapon^{R}N 28⁸ | 35,0 |
| Lamepon^{R}S⁹ | 9,0 |
| Plantaren^{R}1200 | 4,0 |
| Lanette^{R}14¹⁰ | 1,5 |
| Cutina^{R}MD¹¹ | 0,5 |
| Merquat^{R}550¹² | 4,0 |
| Nutrilan^{R}I¹³ | 1,0 |
| Glycerin | 0,5 |
| PHB-Ester | 0,3 |
| Vitamin E-acetat | 0,3 |
| Wasser | ad 100 |

| | |
|---|---|
| ⁸ Laurylethersulfat-Natriumsalz (ca. 28 % Aktivsubstanz; CTFA-Bezeichnung: Sodium Laureth Sulfate) (HENKEL) | |
| ⁹ Eiweißhydrolysat-Fettsäure-Kondensat-Kaliumsalz (CTFA-Bezeichnung: Potassium Cocoyl Hydrolyzed Collagen) (HENKEL) | |
| ¹⁰ Myristylalkohol (CTFA-Bezeichnung: Myristyl Alcohol) (HENKEL) | |
| ¹¹ Palmitinsäure/Stearinsäure-mono/di-glycerid (CTFA-Bezeichnung: Glyceryl Stearate) (HENKEL) | |
| ¹² Dimethyldiallylammoniumchlorid-Acrylamid-Copolymer (8 % Aktivsubstanz in Wasser; CTFA-Bezeichnung: Polyquaternium 7) (MERCK & CO) | |
| ¹³ Kollagenhydrolysat (ca. 39 % Aktivsubstanz; CTFA-Bezeichnung: Hydrolyzed Collagen) (HENKEL) | |

### 5. Shampoo

| | |
|---|---|
| Texapon^{R}N 28 | 36,0 |
| Dehyton^{R}K¹⁴ | 10,0 |
| Plantaren^{R}1200 | 3,0 |
| Lanette^{R}16 | 2,0 |
| Acrylamidopropyltrimethylammoniumchlorid/Acrylsäure(70:30)-Copolymeres, mit Natronlauge neutralisiert | 1,0 |
| Luviskol^{R}K 30¹⁵ | 0,3 |
| Parfümöl | 0,2 |
| Eumulgin^{R}HRE 40 | 0,5 |
| Gluadin^{R}Almond¹⁶ | 0,5 |
| Wasser | ad 100 |

| | |
|---|---|
| ¹⁴ Fettsäureamid-Derivat mit Betainstruktur (ca. 30 % Aktivsubstanz; CTFA-Bezeichnung: Cocoamidopropyl Betaine) (HENKEL) | |
| ¹⁵ Polyvinylpyrrolidon (95 % Aktivsubstanz; CTFA-Bezeichnung: PVP) (BASF) | |
| ¹⁶ Proteinhydrolysat aus Mandelmehl (22 % Aktivsubstanz; CTFA-Bezeichnung: Hydrolyzed Almond Protein) (HENKEL) | |

### 6. mildes Shampoo

| | |
|---|---|
| Lamepon^{R}S | 20,0 |
| Plantaren^{R}2000 | 11,0 |
| Texapon^{R}SB3¹⁷ | 5,0 |
| Dehyton^{R}K | 5,0 |
| Arlypon^{R}F¹⁸ | 2,0 |
| Polymer JR^{R}-400¹⁹ | 0,2 |
| Natriumchlorid | 3,0 |
| Ethylenglykoldistearat | 0,6 |
| Parfümöl | q.s. |
| Farbstoff | q.s. |
| Wasser | ad 100,0 |

| | |
|---|---|
| ¹⁷ Sulfobernsteinsäurehalbester auf Basis eines Alkylpolyglykolethers, Dinatriumsalz (40 % Aktivsubstanz; CTFA-Bezeichnung: Disodium Laurethsulfosuccinate) (HENKEL) | |
| ¹⁸ C_{12/14}-Fettalkohol + 2,5 EO (CTFA-Bezeichnung: Laureth-2) (HENKEL) | |
| ¹⁹ quaternierte Hydroxyethylcellulose (NATIONAL STARCH) | |

### 7. Creme-Pflegeshampoo

| | |
|---|---|
| Cetyl/Stearylalkohol + 20 EO | 1,0 |
| Octyldodecanol | 2,0 |
| Lamecreme DGE 18²⁰ | 4,0 |
| Euperlan PK 3000 | 4,0 |
| Texapon^{R}N70²² | 22,0 |
| Plantaren^{R}2000 | 16,0 |
| Lanette^{R}O²³ | 1,0 |
| Lamequat^{R}L | 1,0 |
| Wasser | ad 100,0 |

| | |
|---|---|
| ²⁰ Polyoxyethylenfettsäurepolyglycerinester (CTFA-Bezeichnung: Polyglyceryl-2-PEG-4-Stearate) (HENKEL) | |
| ²¹ Perlglanzkonzentrat mit Ethylenglykoldistearat (62 % Aktivsubstanz; CTFA-Bezeichnung: Glycol Distearate (and) Glycerin (and) Laureth-4 (and) Cocoamidopropyl Betaine) (HENKEL) | |
| ²² Natriumlaurylethersulfat (72 % Aktivsubstanz; CTFA-Bezeichnung: Sodium Laureth Sulfate) (HENKEL) | |
| ²³ Gemisch höherer, gesättigter Alkohole (CTFA-Bezeichnung: Cetearyl Alcohol) (HENKEL) | |

### 8. Shampoo

| | |
|---|---|
| Lamepon^{R}S | 26,0 |
| Plantaren^{R}1200 | 8,0 |
| Euperlan^{R} PK 3000 | 4,0 |
| Cetiol^{R}HE²⁴ | 2,0 |
| Cosmedia^{R} Guar C 261 | 0,5 |
| Konservierungsmittel | q.s. |
| Farbstoffe | q.s. |
| Parfümöl | q.s. |
| Wasser | ad 100,0 |

| | |
|---|---|
| ²⁴ Polyolfettsäureester (CTFA-Bezeichnung: PEG-7-Glycerylcocoate) (HENKEL) | |

### 9. Shampoo

| | |
|---|---|
| Plantaren^{R}1200 | 4,0 |
| Texapon^{R} N 70 | 4,0 |
| Texapon^{R} SB3 | 2,0 |
| Nutrilan^{R} I | 2,0 |
| Euperlan^{R} PK-789²⁵ | 3,0 |
| Zinkstearat | 0,5 |
| Celquat^{R} H 100²⁶ | 0,3 |
| Konservierungsmittel | q.s. |
| Parfümöl | q.s. |
| Farbstoff | q.s. |
| Wasser | ad 100,0 |

| | |
|---|---|
| ²⁵ Perlglanzkonzentrat (CTFA-Bezeichnung: Sodiumlaureth Sulfate (and) Glycol Distearate (and) Cocoamide MEA) (HENKEL) | |
| ²⁶ quaterniertes Cellulose-Derivat (NATIONAL STARCH) | |

### 10. Shampoo

| | |
|---|---|
| Plantaren^{R}1200 | 4,0 |
| Texapon^{R} N 70 | 4,0 |
| Texapon^{R} SB3 | 2,0 |
| Nutrilan^{R} I | 2,0 |
| Euperlan^{R} PK-789 | 3,0 |
| Aluminiumstearat | 0,5 |
| Celquat^{R} H 100 | 0,3 |
| Konservierungsmittel | q.s. |
| Parfümöl | q.s. |
| Farbstoff | q.s. |
| Wasser | ad 100,0 |

## Patentansprüche

1. Verwendung eines wäßrigen Mittels, gekennzeichnet durch einen Gehalt an
a) Polymeren (A), ausgewählt aus der Gruppe der kationischen, amphoteren, zwitterionischen und nichtionischen Polymeren,
b) Alkylpolyglykosiden (B) der allgemeinen Formel (I)
RO-(Z)ₓ (I)
in denen
R steht für einen Alkylrest mit 6 bis 22 Kohlenstoffatomen,
Z für einen Mono- oder Oligosaccharid,
x für eine Zahl von 1-1 bis 5,
oder deren Anlagerungsprodukten mit 1 bis 10 Molekülen Ethylenoxid
und/oder Propylenoxid sowie
c) Fettalkoholen als Fettstoffe (C)
zum Reinigen oder Pflegen von Keratinfasern, insbesondere menschlichen Haaren.

2. Verwendung nach Anspruch 1, dadurch gekennzeichnet, daß das wäßrige Mittel
- 0,1 bis 3 Gew.-% an Polymeren (A)
- 0,01 bis 10 Gew.-% an Alkylpolyglykosiden (B) und
- 0,5 bis 20 Gew.-% an Fettalkoholen (C),
jeweils bezogen auf das gesamte Mittel, enthält.

3. Verwendung nach einem der Ansprüche 1 oder 2, dadurch gekennzeichnet, daß das Polymer (A) ausgewählt ist aus der Gruppe der amphoteren und zwitterionischen Polymeren.

4. Verwendung nach einem der Ansprüche 1 bis 3, dadurch gekennzeichnet, daß das Polymer (A) ein zwitterionisches Polymer, aufgebaut aus mindestens einem Monomertyp mit kationischem Charakter und mindestens einem Monomertyp mit anionischem Charakter, ist.

5. Verwendung nach einem der Ansprüche 1 bis 4, dadurch gekennzeichnet, daß in der Formel (I) Z für Glucose und x für eine Zahl zwischen 1,1 und 1,6, insbesondere für eine Zahl zwischen 1,1 und 1,4 steht.

6. Verwendung nach einem der Ansprüche 1 bis 5, dadurch gekennzeichnet, daß das Zuckerderivat (B) in einer Menge von 0,1 - 0,9 Gew.-%, bezogen auf das gesamte Mittel, enthalten ist.

7. Verwendung nach einem der Ansprüche 1 bis 6, dadurch gekennzeichnet, daß das Mittel frei ist von kationischen und anionischen Tensiden.

8. Verwendung nach einem der Ansprüche 1 bis 7, dadurch gekennzeichnet, daß das Mittel als Lotion, Emulsion oder Mikroemulsion mit einem Wassergehalt von 50 - 90 Gew.-%, bezogen auf das gesamte Mittel, vorliegt.

9. Verwendung nach einem der Ansprüche 1 bis 8, dadurch gekennzeichnet, daß das Mittel zur Verdickung einen ethoxylierten Fettalkohol mit eingeengter Homologenverteilung und/oder einen Ester aus einem ethoxylierten Polyol und Fettsäuren enthält.

10. Verwendung nach einem der Ansprüche 1 bis 9 zur Nachbehandlung von Haaren.

## Claims

1. The use of a water-based formulation, which is characterized by a content of
a) polymers (A) selected from the group of cationic, amphoteric, zwitterionic and nonionic polymers,
b) alkyl polyglycosides (B) corresponding to general formula (I):
RO-(Z)ₓ (I) (I)
in which
R is an alkyl radical containing 6 to 22 carbon atoms,
Z is a mono- or oligosaccharide,
x is a number of 1.1 to 5,
or adducts thereof with 1 to 10 molecules of ethylene oxide and/or propylene oxide and
c) fatty alcohols as fatty compounds (C),
for the cleaning and care of keratin fibres, more particularly human hair.

2. The use claimed in claim 1, characterized in that the water-based formulation contains
- 0.1 to 3% by weight of polymers (A)
- 0.01 to 10% by weight of alkyl polyglycosides (B) and
- 0.5 to 20% by weight of fatty alcohols (C),
based in each case on the formulation as a whole.

3. The use claimed in claim 1 or 2, characterized in that the polymer (A) is selected from the group of amphoteric and zwitterionic polymers.

4. The use claimed in any of claims 1 to 3, characterized in that the polymer (A) is a zwitterionic polymer made up of at least one cationic monomer and at least one anionic monomer.

5. The use claimed in any of claims 1 to 4, characterized in that, in formula (I), Z is glucose and x is a number of 1.1 to 1.6 and, more particularly, a number of 1.1 to 1.4.

6. The use claimed in any of claims 1 to 5, characterized in that the sugar derivative (B) is present in a quantity of 0.1 to 0.9% by weight, based on the formulation as a whole.

7. The use claimed in any of claims 1 to 6, characterized in that the formulation is free from cationic and anionic surfactants.

8. The use claimed in any of claims 1 to 7, characterized in that the formulation is formulated as a lotion, emulsion or microemulsion with a water content of 50 to 90% by weight, based on the formulation as a whole.

9. The use claimed in any of claims 1 to 8, characterized in that, for thickening, the formulation contains a narrow-range ethoxylated fatty alcohol and/or an ester of an ethoxylated polyol and fatty acids.

10. The use claimed in any of claims 1 to 9 for the aftertreatment of hair.

## Revendications

1. Utilisation d'un produit aqueux, caractérisé par un contenu en
a) polymères (A), sélectionnés parmi le groupe des polymères cationiques, amphotères, zwitterioniques et non ioniques,
b) alkylpolyglycosides (B) de la formule générale (I)
RO-(Z)ₓ (I) (I)
dans laquelle
R représente un radical alkyle comportant 6 à 22 atomes de carbone,
Z correspond à un mono- ou à un oligosaccharide
x est un nombre de 1,1 à 5,
ou leurs produits d'addition avec 1 à 10 molécules d'oxyde d'éthylène et/ou d'oxyde de propylène, ainsi que
c) des alcools gras comme matières grasses (C)
pour le lavage ou le soin des fibres de kératine, en particulier de la chevelure humaine.

2. Utilisation selon la revendication 1, caractérisée en ce que le produit aqueux renferme
- 0,1 à 3 % en poids de polymères (A),
- 0,01 à 10 % en poids d'alkylpolyglycosides (B) et
- 0,5 à 20 % en poids d'alcools gras (C),
dans chaque cas par rapport à la totalité du produit.

3. Utilisation selon une des revendications 1 ou 2, caractérisée en ce que le polymère (A) est sélectionné parmi le groupe des polymères amphotères et zwitterioniques.

4. Utilisation selon une des revendications 1 à 3, caractérisée en ce que le polymère (A) est un polymère zwitterionique, constitué d'au moins un type de monomère à caractère cationique et d'au moins un type de monomère à caractère anionique.

5. Utilisation selon une des revendications 1 à 4, caractérisée en ce que, dans la formule (I), Z représente le glucose et X est un nombre compris entre 1,1 et 1,6, en particulier un nombre situé entre 1,1 et 1,4.

6. Utilisation selon une des revendications 1 à 5, caractérisée en ce que le dérivé de sucre (B) est contenu dans une proportion de 0,1 à 0,9 % en poids, par rapport à la totalité du produit.

7. Utilisation selon une des revendications 1 à 6, caractérisée en ce que le produit est exempt de tensioactifs cationiques et anioniques.

8. Utilisation selon une des revendications 1 à 7, caractérisée en ce que le produit est formulé comme lotion, émulsion ou micro-émulsion avec une teneur en eau de 50 à 90 % en poids par rapport à la totalité du produit.

9. Utilisation selon une des revendications 1 à 8, caractérisée en ce que le produit renferme pour l'épaissir, un alcool gras éthoxylé à répartition resserrée des homologues et/ou un ester d'un polyol éthoxylé et d'acides gras.

10. Utilisation selon une des revendications 1 à 9 pour le traitement postérieur des cheveux.
